# EUROPEAN PATENT APPLICATION

(11) **EP 3 892 361 A1**
(43) Date of publication of application: **13.10.2021**
(21) Application number: 20706050.0
(22) Date of filing: 25.02.2020
(51) Int. Cl.: B01D 53/88, B01J 35/00, A61L 9/20, F24F 3/16, B01D 46/00, B01D 46/42, F21K 9/237

(54) **PHOTOCATALYST FILTER MODULE AND AIR PURIFIER INCLUDING SAME**

(30) Priority: 11.02.2020 KR 20200016267
(71) Applicant: ACE ONE CO., LTD., Gwangsan-gu, Gwangju 62464 (KR)
(72) Inventor: KIM, Bo Hyun, Gwangju 62000 (KR); LEE, Dae Young, Gwangju 62385 (KR); KANG, Ji Hun, Gwangju 62299 (KR); KIM, Sung Rok, Gwangju 62000 (KR)
(74) Representative: Isarpatent
(86) International application number: PCT/KR2020/002692
(87) International publication number: WO 2021/162167

(57) **Abstract**

The present invention provides a photocatalytic filter module and an air purifier. The present invention includes a base frame in which at least one side is open, a plurality of support frames arranged to traverse the base frame, a plurality of light emitting units arranged on the support frame, and filter units installed in the base frame and disposed to be spaced apart from the light emitting units.

## Description

### [Technical Field]

The present invention relates to a photocatalytic filter module and an air purifier including the same.

### [Background Art]

Since harmful ingredients such as heavy metal and the like among a variety of foreign substances and the like have recently flowed in on the westerlies from China and also fine dust has a size too small to be prevented from flowing indoors through gaps in the windows and has a very bad influence on health, a filtration process of fine dust becomes a very important issue such that air purifiers have generally become widespread in homes, offices, or the like.

To describe in more detail, dust floating in the air such as particulate matter (PM) and dust particles is atmosphere pollution matter including a large number of air pollutants in addition to a sulfurous acid gas, nitrogen oxide, lead, ozone, carbon monoxide, and the like. Here, fine dust having a particle diameter of 10 µm or less which is generated by vehicles, plants, and the like and floats in the air for a long time is referred to as PM₁₀. Among PM₁₀ particles, particles of 2.5 µm or less are designated as PM_{2.5} and called ultra-particulate matter and academically referred to collectively as aerosol. Fine particles are called suspended particles, particulate matter, or the like. According to designations, meanings thereof differ slightly.
The particulate matter has an aerodynamic particle diameter of 10 nm to 100 µm. Particles having a particle diameter greater than this are not highly problematic because a retention time thereof in the air is very short due to a sinking effect caused by gravity. However, fine particles, particularly, ultrafine particles, stay in the air for a long time, freely move according to movement of an air current, have a particle size too small to be filtered using a general filter such as to have a negative influence on the elderly with weak immunity, pregnant women, and fetuses and cause asthma, headaches, and atopy, and to increase blood sugar by increasing insulin resistance which have been reported in medical circles as having a risk of causing a metabolic syndrome, cardiac disease, diabetes, and the like.

As demand for air purifiers increases, a demand for air purifiers installed in a small space such as a vehicle and the like and configured to effectively purify air is also increasing. Needs for air purifiers having a compact size to increase portability and space utilization and configured to be simply maintained and repaired by easily replacing a filter are increasing.

### [Disclosure]

### [Technical Problem]

The present invention is directed to providing a photocatalytic filter module capable of effectively purifying the air and an air purifier including the same. However, such an aspect is merely an example and is not intended to limit the scope of the present invention.

### [Technical Solution]

One aspect of the present invention provides a photocatalytic filter module including a base frame in which at least one side is open, a plurality of support frames disposed to traverse the base frame, a plurality of light emitting units arranged on the support frame, and filter units installed in the base frame and disposed to be spaced apart from the light emitting units.

When the light emitting unit emits light toward the filter units, the filter units may be activated and sterilize a gas passing through the base frame.

The plurality of support frames may be arranged to be spaced apart from each other and form an open area.

The plurality of light emitting units may be arranged to be spaced apart along a longitudinal direction of the support frames.

The filter units may be disposed above the support frames to be spaced apart therefrom and intersect with the support frames.

The filter units may be disposed to be inclined with respect to the support frames.

An inclination angle of the filter units may be set to be any one within a range of 15 degrees to 75 degrees.

A minimum distance from the filter units to the support frames may be set to be any one within a range of 1 mm to 10 mm.

The light emitting units may be disposed on both sides of the support frames, and the filter units may be disposed to be spaced apart from both of the sides of the support frames to correspond to the light emitting units.

The filter unit may be formed to have a flat panel shape or to be at least partially curved.

Another aspect of the present invention provides an air purifier including a housing having an inlet and an outlet, a photocatalytic filter module disposed in an internal space of the housing, and a fan unit configured to discharge a gas which has passed through the photocatalytic filter module through the outlet. Here, the photocatalytic filter module includes a base frame in which at least one side is open, a plurality of support frames arranged to traverse the base frame, a plurality of light emitting units arranged on the support frame, and filter units installed in the base frame and disposed to be spaced apart from the light emitting units.

Other aspects, features, and advantages in addition to the above description will be apparent from the detailed description, the claims, and the drawings of the present invention.

### [Advantageous Effects]

A photocatalytic filter module and an air purifier according to one embodiment of the present invention may purify and sterilize an external gas which flows thereinto. A gas which moves inward may be purified by a filter portion and may be discharged outward after being purified or sterilized by the photocatalytic filter module. The photocatalytic filter module includes light emitting units and filter units arranged on at least one side thereof so as to increase efficiency in purifying and sterilizing a gas.

The photocatalytic filter module and the air purifier according to one embodiment of the present invention may increase efficiency of purifying a gas. Since the filter units are arranged to be spaced at a preset distance apart from the light emitting units and have a certain inclination angle, a moving gas may be in contact with the filter units for a long time. Since the filter units are disposed to be inclined, a contact area and time with the gas may be increased so as to improve air purification efficiency.

The photocatalytic filter module and the air purifier according to one embodiment of the present invention may be activated by the light emitting units so as to increase a service time and so as to sterilize a discharged gas. Also, since the photocatalytic filter module according to one embodiment of the present invention can be manufactured to be compact, it is possible to provide the air purifier which is miniaturized. However, the scope of the present invention is not limited by the above effects.

### [Description of Drawings]

FIG. 1 is an exploded perspective view of an air purifier according to one embodiment of the present invention.
FIG. 2 is a perspective view illustrating a photocatalytic filter module of FIG. 1.
FIG. 3 is a cross-sectional view taken along line III-III of FIG. 2.
FIG. 4 is a perspective view illustrating a photocatalytic filter module according to another embodiment of the present invention.
FIG. 5 is a cross-sectional view taken along line V-V of FIG. 4.
FIG. 6 is a perspective view illustrating a modified example of a filter unit of FIG. 2.

### [Modes of the Invention]

Reference will now be made in detail to the exemplary embodiments which are described in reference to the accompanying drawings, wherein like reference numerals refer to like elements throughout. In this regard, the embodiments may have different forms and should not be construed as being limited to the descriptions set forth herein. Accordingly, the embodiments are merely described below, by referring to the figures, to explain aspects of the embodiments. As used herein, the term "and/or" includes any and all combinations of one or more of the associated listed items. Expressions such as "at least one of," when preceding a list of elements, modify the entire list of elements and do not modify the individual elements of the list.

Since the inventive concept may have various modifications and several embodiments, exemplary embodiments are shown in the drawings and will be described in detail. Advantages, features, and a method of achieving the same will be specified with reference to the embodiments described below in detail together with the attached drawings. However, the embodiments may have different forms and should not be construed as being limited to the descriptions set forth herein.

It will be understood that although the terms "first", "second", etc. may be used herein to describe various components, these components should not be limited by these terms. These components are only used to distinguish one component from another.

Singular expressions, unless defined otherwise in contexts, include plural expressions.

In the embodiments below, it will be further understood that the terms "comprise" and/or "have" used herein specify the presence of stated features or components, but do not preclude the presence or addition of one or more other features or components.

In the embodiments below, it will be understood when a portion such as a layer, an area, or an element is referred to as being "on" or "above" another portion, it can be directly on or above the other portion, or intervening portion may also be present.

Also, in the drawings, for convenience of description, sizes of elements may be exaggerated or contracted. In other words, since sizes and thicknesses of components in the drawings are arbitrarily illustrated for convenience of explanation, the following embodiments are not limited thereto.

The exemplary embodiments should be considered in descriptive sense only and not for purposes of limitation. Descriptions of features or aspects within each embodiment should typically be considered as available for other similar features or aspects in other embodiments.

While the present invention has been particularly shown and described with reference to exemplary embodiments thereof, it will be understood by those of ordinary skill in the art that various changes in form and details may be made therein without departing from the spirit and scope of the present invention as defined by the following claims.

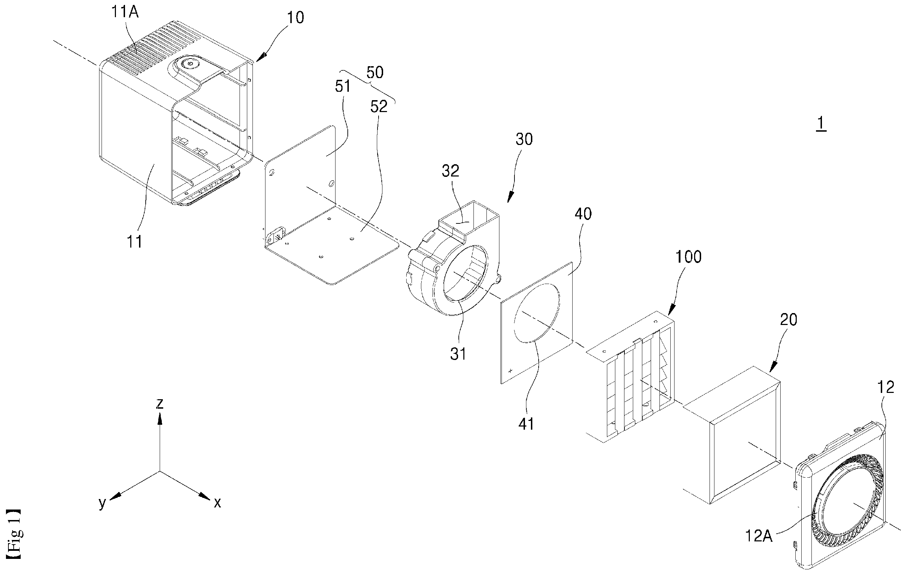

FIG. 1 is an exploded perspective view of an air purifier 1 according to one embodiment of the present invention.

Referring to FIG. 1, the air purifier 1 may circulate a gas, particularly, air, and purify the air at a filter thereof. The air purifier 1 may safely purify a gas with high efficiency using a photocatalytic filter. The air purifier 1 may include a housing 10, a filter member 20, a fan unit 30, a connection plate 40, a bracket 50, and a photocatalytic filter module 100.

The housing 10 may form an exterior of the air purifier 1 and may have an inlet 12A and an outlet 11A. In an internal space of the housing 10, the filter member 20, the fan unit 30, the connection plate 40, the bracket 50, and the photocatalytic filter module 100 may be disposed.

The housing 10 may have a variety of shapes. Although a cubic shape is shown in the drawing, the housing is not limited thereto and may have a variety of shapes such as a cuboid shape, a spherical shape, a polyprism shape, a cylindrical shape, and the like. However, hereinafter, for convenience of description, an embodiment in which the housing 10 is a cube formed by assembling a first cover 11 with a second cover 12 will be mainly described.

The first cover 11 has an internal space and a front thereof is open. The first cover 11 includes the outlet 11A such that a gas may be discharged outward again. The outlet 11A is disposed above the first cover 11 in FIG. 1 but is not limited thereto, and a position of the outlet 11A may be variously set.

The second cover 12 may be assembled with the front of the first cover 11 and may have the inlet 12A. The inlet 12A has a circular shape in a center of the second cover 12 in FIG. 1 but a position and a shape of the inlet 12A may be variously set in consideration of a flow of a gas.

The filter member 20 may be disposed in the internal space of the housing 10 and primarily filter a gas inflow. The filter member 20 may be disposed at a rear end of the second cover 12 and filter a gas which flows thereinto through the inlet 12A.

The filter member 20 may filter out foreign substances, particularly, fine dust and the like, which flow thereinto with a gas. As an example, the filter member 20 may be a high efficiency particular air (HEPA) filter. The filter member 20 may be installed to be mounted or demounted by opening the housing 10 so as to be replaceable after the air purifier 1 is used for a certain period.

The fan unit 30 may be installed in the internal space of the housing 10 and may discharge a purified gas through the outlet 11A. The fan unit 30 may discharge a gas, which has passed through the photocatalytic filter module 100, through the outlet 11A. The fan unit 30 is an air blower which generates a gas flow and may include an inlet portion 31 through which the gas, which has passed through the photocatalytic filter module 100, flows and may include an outlet portion 32 disposed to face the outlet 11A.

The connection plate 40 may be selectively provided in the air purifier 1. The connection plate 40 may include an opening 41 which faces the inlet portion 31 of the fan unit 30 and be disposed between the photocatalytic filter module 100 and the fan unit 30.

In one embodiment, the bracket 50 may support at least one of the filter member 20, the fan unit 30, and the photocatalytic filter module 100. Since at least one of the filter member 20, the fan unit 30, and the photocatalytic filter module 100 is mounted on the bracket 50, a user may replace an internal component by replacing the bracket 50.

The bracket 50 may include a first sidewall 51 and a second sidewall 52, and the first sidewall 51 and the second sidewall 52 are formed to be bent. The first sidewall 51 may be mounted in the rear of the internal space of the housing 10, and the second sidewall 52 may be mounted on a bottom of the internal space of the housing 10.

As another embodiment, an air purifier may be installed without a bracket. That is, the filter member 20, the fan unit 30, the connection plate 40, and the photocatalytic filter module 100 may be directly mounted in the housing 10.

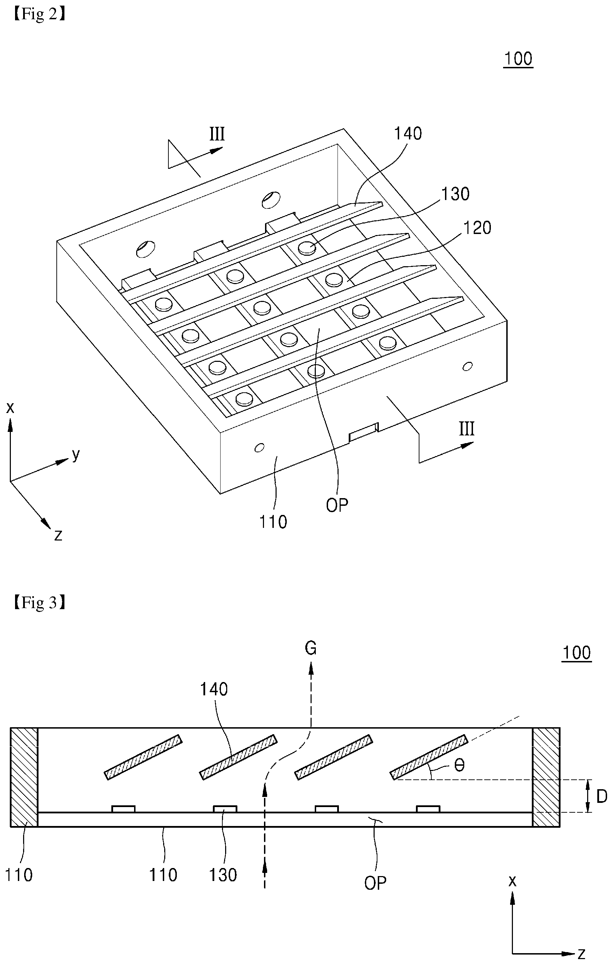

FIG. 2 is a perspective view illustrating the photocatalytic filter module 100 of FIG. 1, and FIG. 3 is a cross-sectional view taken along line III-III of FIG. 2.

Referring to FIGS. 1 to 3, the photocatalytic filter module 100 may be disposed in the internal space of the housing 10. The photocatalytic filter module 100 may be activated by light and purify a gas passing through the internal space. The photocatalytic filter module 100 may include a base frame 110, support frames 120, light emitting units 130, and filter units 140.

As one embodiment, the photocatalytic filter module 100 may be disposed between the filter member 20 and the fan unit 30. The photocatalytic filter module 100 may purify the gas primarily filtered by the filter member 20.

The base frame 110 may be disposed such that one side thereof is open, and the gas may pass through an open part. The base frame 110 may be variously formed according to a shape of the housing 10. Additionally, the base frame 110 may be disposed such that both sides thereof are open. Since the base frame 110 is supported by an inner wall of the housing 10, a shape thereof may be set to correspond to an inner shape of the housing 10. As an example, the base frame 110 may have a quadrangular frame shape.

A plurality of such support frames 120 may be disposed to traverse the base frame 110. The plurality of support frames 120 may be arranged to be spaced apart from each other and form an open area OP.

The gas, which has passed through the filter member 20, may flow into the open area OP and move toward the filter unit 140. That is, the open area OP may form a space in the photocatalytic filter module 100 in which the gas is movable.

The number of the support frames 120 is not limited to a particular number and may be variously set according to a size of the photocatalytic filter module 100. As an example, as shown in FIG. 2, three support frames 120 may be arranged to be spaced apart from one another.

The light emitting unit 130 may be installed on the support frame 120. The light emitting unit 130 may be disposed on at least one surface of the support frame 120. The light emitting units 130 may be arranged in a row along a longitudinal direction of the support frame 120. The support frame 120 has a stick shape having a certain thickness in FIG. 3 but is not limited thereto and may have a variety of shapes.

The light emitting units 130 may be arranged on the support frame 120. The light emitting units 130 may be set as a plurality of lamps. A plurality of such light emitting units 130 may be arranged to be spaced apart from each other along the longitudinal direction of the support frame 120.

The light emitting units 130 may emit light of a wavelength band which activates the filter unit 140. When the light emitting unit 130 is driven, the filter unit 140, which faces the light emitting units 130, may be activated and purify a moving gas.

As one embodiment, a lamp included in the light emitting unit 130 may be a light emitting diode (LED) lamp. The LED lamp may be mounted on one surface of the support frame 120.

The light emitting unit 130 may be disposed to face the filter unit 140. Such lamps included in the light emitting unit 130 may be arranged to face the filter units 140. Referring to FIG. 3, the light emitting units 130 are arranged to correspond to the filter units 140.

As an example, when the light emitting unit 130 emits light of any one of wavelength bands of visible rays, infrared rays, and ultraviolet rays, the filter units 140 are activated.

As another example, the light emitting unit 130 may emit light of a plurality of wavelength bands. Some of a plurality of such lamps may emit light of a wavelength band of visible rays, others may emit light of a wavelength band of infrared rays, and still others may emit light of a wavelength band of ultraviolet rays.

As another example, a controller (not shown) may adjust a wavelength band of light emitted by the light emitting unit 130. For example, the wavelength band of the light emitted by the light emitting unit 130 may be set by the controller.

The filter unit 140 is disposed to be spaced at a certain distance D apart from the light emitting unit 130 and is installed to be supported by the base frame 110. The filter unit 140 may be activated by the light emitted by the light emitting unit 130.

As one embodiment, when the filter unit 140 is activated, the gas passing through the filter unit 140 may be sterilized. When the light emitting unit 130 emits light toward the filter unit 140, the filter unit 140 may be activated and sterilize the gas passing through the base frame 110.

As another embodiment, when the filter unit 140 is activated, foreign substances in the gas passing through the filter unit 140 may be filtered out again. As still another embodiment, when the filter unit 140 is activated, a scent may be added or negative ions may be added to the gas passing through the filter unit 140.

As one embodiment, the filter unit 140 may be formed to have an approximate panel shape having a certain thickness. The filter unit 140 may have a flat panel shape and purify or sterilize a gas which comes into contact with the filter unit 140.

A plurality of such filter units 140 may be arranged to be spaced apart. The plurality of filter units 140 are arranged above the support frames 120 to be spaced apart and to intersect with the support frames 120. Referring to FIG. 3, the support frames 120 are arranged in parallel along a Z-axis direction, and the filter units 140 are arranged in parallel along a Y-axis direction. Since the support frames 120 and the filter units 140 are arranged in different directions from each other, a contact area of a gas passing through the open area OP of the support frames 120 increases such that efficiency of the filter units 140 may be increased.

The filter units 140 may be arranged to be inclined with respect to the support frames 120. An inclination angle θ of the filter units 140 may be set to be any one within a range of 15 degrees to 75 degrees. When the inclination angle θ of the filter units 140 is smaller than 15 degrees, a path through which a gas is movable is blocked such that flow efficiency is degraded. Also, when the inclination angle θ of the filter units 140 is greater than 75 degrees, a contact area between the gas G and the filter units 140 decreases such that efficiency of the filter units 140 decreases.

Since the filter units 140 have the inclination angle θ within a range of 15 degrees to 75 degrees with respect to the support frames 120, efficiency of purifying the moving gas G may be increased. Preferably, the inclination angle θ may be set to be any one within a range of 20 degrees to 45 degrees. Particularly, the inclination angle θ may be set to be about 27 degrees.

As another embodiment, the inclination angle of the filter units may be adjusted. The user may adjust the inclination of the filter units in consideration of a flow rate of a gas.

The filter units 140 may be arranged to be spaced at a certain distance D apart from the support frames 120. A minimum distance D between the filter units 140 and the support frames 120 may be set to be any one within a range of 1 mm to 10 mm. When the distance D is smaller than 1 mm, a space in which the gas G is movable is not secured. When the distance D is greater than 10 mm, a distance between the lamps and the filter units 140 is far such that it is difficult to activate the filter units 140. The filter units 140 are spaced apart at the distance which is any one within the range of 1 mm to 10 mm apart from the support frames 120 such that the filter units 140 may be activated with high efficiency when the light emitting units 130 emit light. Preferably, the distance D may be set to be any one within a range of 3 mm to 8 mm. Particularly, the distance D may be set to be about 5 mm.

The photocatalytic filter module 100 and the air purifier 1 according to one embodiment of the present invention may purify and sterilize an external gas which flows thereinto. The gas which moves thereinto may be purified by the filter member 20, be purified or sterilized by the photocatalytic filter module 100, and then be discharged outward.

The photocatalytic filter module 100 and the air purifier 1 according to one embodiment of the present invention may increase efficiency of purifying a gas. Since the filter units 140 are arranged to be spaced at a preset distance D apart from the light emitting units 130 and have a certain inclination angle θ, a moving gas may be in contact with the filter units 140 for a long time. Since the filter units 140 are disposed to be inclined, a contact area and time with the gas may be increased so as to improve air purification efficiency.

The photocatalytic filter module 100 and the air purifier 1 according to one embodiment of the present invention may be activated by the light emitting units 130 so as to increase a service time and to sterilize a discharged gas. Also, since the photocatalytic filter module 100 according to one embodiment of the present invention can be manufactured to be compact, it is possible to provide the air purifier 1 which is miniaturized.

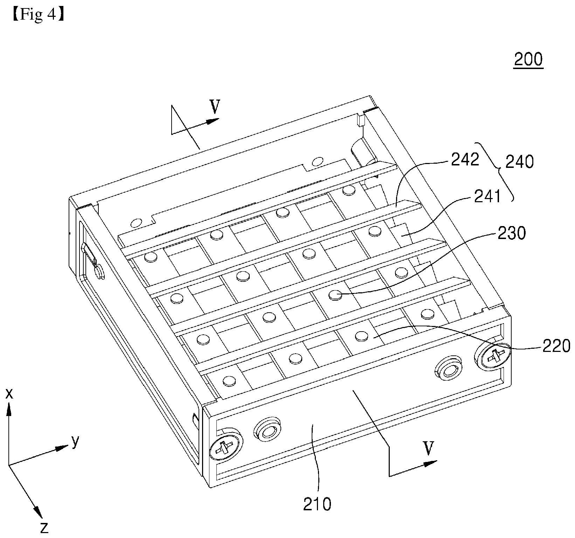

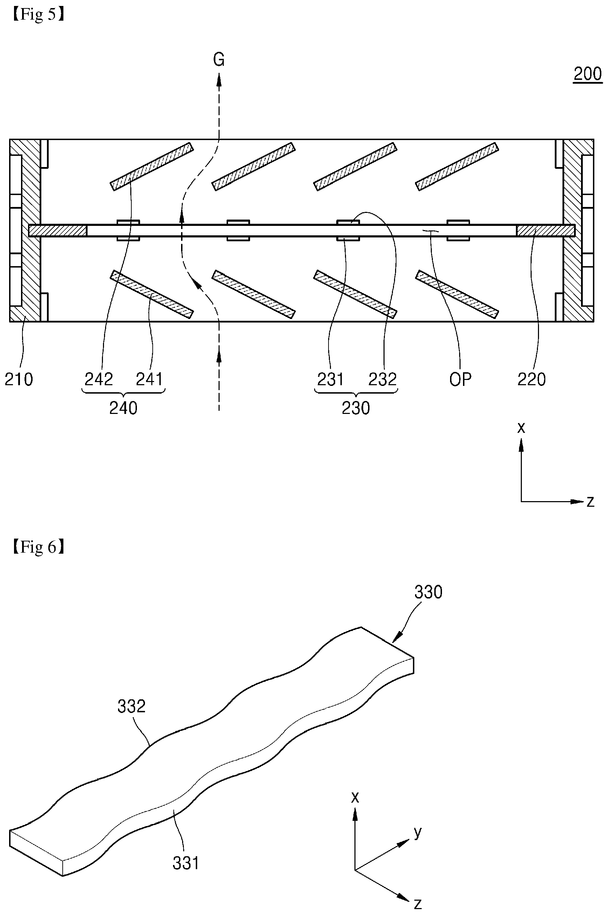

FIG. 4 is a perspective view illustrating a photocatalytic filter module 200 according to another embodiment of the present invention, and FIG. 5 is a cross-sectional view taken along line V-V of FIG. 4.

Referring to FIGS. 4 and 5, in the photocatalytic filter module 200, light emitting units 230 and filter units 240 are arranged on each of both sides, that is, in an inlet and an outlet of the photocatalytic filter module 200. The photocatalytic filter module 200 may include a base frame 210, support frames 220, the light emitting units 230, and the filter units 240.

The base frame 210 may be disposed such that one side is open, and a gas may pass through an open part. The base frame 210 may have two sides which are open. Accordingly, a gas which flows in through the inlet may be discharged toward an outlet portion.

The support frames 220 may be arranged to traverse the base frame 210. The light emitting units 230 may be arranged on the support frame 220. The support frames 220 may be arranged between an inlet portion and an outlet portion of the base frame 210. As one embodiment, the support frames 220 may be arranged in the middle of the base frame 210 in a thickness direction.

The support frames 220 form an open area OP, and a gas G may pass through the open area OP and move from a front to a rear of the photocatalytic filter module 200.

The light emitting units 230 may be arranged on both sides of the support frames 220. The light emitting units 230 may include first lamps 231 disposed on one surface of the support frame 220 and second lamps 232 disposed on the other surface.

The first lamp 231 may be arranged to face an inlet side of the photocatalytic filter module 200 and emit light toward a first filter portion 241. The second lamp 232 may be arranged to face an outlet side of the photocatalytic filter module 200 and emit light toward a second filter portion 242.

As one embodiment, the first lamps 231 and the second lamps 232 may emit light of the same wavelength band. For example, both the first filter portion 241 and the second filter portion 242 may perform the same sterilization function or minute purification function.

As another embodiment, the first lamp 231 and the second lamp 232 may emit light of different wavelength bands so as to allow the first filter portion 241 and the second filter portion 242 to perform different functions. For example, the first filter portion 241 may perform a sterilization function, and the second filter portion 242 may perform a minute purification function.

The filter units 240 may be arranged on both sides of the support frames 220 to be spaced apart to correspond to the light emitting units 230. The filter unit 240 may include the first filter portion 241 disposed on the inlet side of the photocatalytic filter module 200 and the second filter portion 242 disposed on the outlet side of the photocatalytic filter module 200.

The filter unit 240 may be disposed to have a certain inclination and to be spaced apart from a surface of the support frame 220. The first filter portion 241 may be disposed to be spaced apart from the support frame 220 and to have a certain inclination. The second filter portion 242 may be disposed to be spaced apart from the support frame 220 and to have a certain inclination.

As one embodiment, as shown in FIG. 5, the first filter portion 241 and the second filter portion 242 may have inclinations in different directions. That is, the first filter portion 241 may have an inclination to tilt downward in a +Z axis direction, and the second filter portion 242 may have an inclination to tilt downward in a -Z axis direction.

Since the first filter portion 241 and the second filter portion 242 have inclinations in different directions, a contact time of the filter unit 240 and the gas may be increased. That is, since the gas G moves between the first filter portions 241, passes through the open area OP, and passes between the second filter portions 242 in parallel in an opposite direction, a staying time in the photocatalytic filter module 200 may be increased.

As another embodiment, the first filter portion and the second filter portion may have inclinations in the same direction. Since the first filter portion and the second filter portion are arranged in parallel in the same direction, fluidity of the gas may be increased so as to increase air circulation efficiency.

The first filter portions 241 and the second filter portions 242 may have different sizes or differ in numbers. In consideration of the fluidity of the gas G, the sizes or numbers of the first filter portions 241 and the second filter portions 242 may be set to be different.

The photocatalytic filter module 200 and the air purifier according to one embodiment of the present invention may purify and sterilize an external gas which flows thereinto. The gas which moves thereinto may be purified by the filter member 20, be purified or sterilized by the photocatalytic filter module 200, and then be discharged outward. The light emitting units 230 and the filter units 240 are arranged on both the inlet side and the outlet side of the photocatalytic filter module 200 so as to increase efficiency of purifying and sterilizing a gas.

The photocatalytic filter module 200 and the air purifier according to one embodiment of the present invention may increase efficiency of purifying a gas. Since the filter units 240 are arranged to be spaced at a preset distance D apart from the light emitting units 230 and have a certain inclination angle θ, a gas which moves may be in contact with the filter units 240 for a long time. Since the filter units 240 are disposed to be inclined, a contact area and contact time with the gas may be increased so as to improve air purification efficiency.

The photocatalytic filter module 200 and the air purifier according to one embodiment of the present invention may be activated by the light emitting units 230 so as to increase a service time and to sterilize a discharged gas. Also, since the photocatalytic filter module 200 according to one embodiment of the present invention can be manufactured to be compact, it is possible to provide an air purifier 2 which is miniaturized.

FIG. 6 is a perspective view illustrating a modified example of the filter unit 140 of FIG. 2.

Referring to FIG. 6, a filter unit 330 may be formed to be at least partially curved. The filter unit 330 may be formed such that a first side surface 331 and a second side surface 332 are curved. Since the first side surface 331 and the second side surface 332 have a waved shape, the gas G may smoothly pass through the filter unit 330. Also, since the first side surface 331 and the second side surface have the waved shape, fluidity of the gas G in the photocatalytic filter module is complexly formed and a contact amount and contact time of the filter unit 330 and the gas may increase so as to increase a filtering effect.

Although the embodiments of the present invention have been described with reference to the drawings, the embodiments are merely examples and it should be understood by one of ordinary skill in the art that a variety of modifications and equivalents thereof may be made therefrom. Accordingly, the technical scope of the present invention should be determined by the technical concept of the following claims.

### [Description of Reference Numerals]

- 1:: air purifier
- 10:: housing
- 20:: filter member
- 30:: fan unit
- 40:: connection plate
- 50:: bracket
- 100, 200:: photocatalytic filter module

## Claims

1. A photocatalytic filter module comprising:
a base frame in which at least one side is open;
a plurality of support frames arranged to traverse the base frame;
a plurality of light emitting units arranged on the support frame; and
filter units installed in the base frame and disposed to be spaced apart from the light emitting units.

2. The photocatalytic filter module of claim 1, wherein when the light emitting unit emits light toward the filter units, the filter units are activated and sterilize a gas passing through the base frame.

3. The photocatalytic filter module of claim 1, wherein the plurality of support frames are arranged to be spaced apart from each other and form an open area.

4. The photocatalytic filter module of claim 1, wherein the plurality of light emitting units are arranged to be spaced apart along a longitudinal direction of the support frames.

5. The photocatalytic filter module of claim 1, wherein the filter units are disposed above the support frames to be spaced apart therefrom and intersect with the support frames.

6. The photocatalytic filter module of claim 1, wherein the filter units are disposed to be inclined with respect to the support frames.

7. The photocatalytic filter module of claim 6, wherein an inclination angle of the filter units is set to be any one within a range of 15 degrees to 75 degrees.

8. The photocatalytic filter module of claim 1, wherein a minimum distance from the filter units to the support frames is set to be any one within a range of 1 mm to 10 mm.

9. The photocatalytic filter module of claim 1, wherein the light emitting units are disposed on both sides of the support frames, and
wherein the filter units are disposed to be spaced apart from the both sides of the support frames to correspond to the light emitting units.

10. The photocatalytic filter module of claim 1, wherein the filter units are formed to have a flat panel shape or to be at least partially curved.

11. An air purifier comprising:
a housing having an inlet and an outlet;
a photocatalytic filter module disposed in an internal space of the housing; and
a fan unit configured to discharge a gas which has passed through the photocatalytic filter module through the outlet,
wherein the photocatalytic filter module comprises:
a base frame in which at least one side is open;
a plurality of support frames arranged to traverse the base frame;
a plurality of light emitting units arranged on the support frame; and
filter units installed in the base frame and disposed to be spaced apart from the light emitting units.
